# EUROPEAN PATENT APPLICATION

(11) **EP 3 964 182 A1**
(43) Date of publication of application: **09.03.2022**
(21) Application number: 21175968.3
(22) Date of filing: 26.05.2021
(51) Int. Cl.: A61F 5/445, A61M 16/04

(54) **APPLICATOR FOR TRACHEOSTOMA BASEPLATE**

(30) Priority: 04.09.2020 GB 202013922
(71) Applicant: Kapitex Healthcare Limited, Wetherby West Yorkshire LS22 7DN (GB)
(72) Inventor: WORTHINGTON, Ian David, Wetherby, LS22 7DN (GB); BRIERLEY, Alexander James, Wetherby, LS22 7DN (GB)
(74) Representative: Finch, Steven Caffall

(57) **Abstract**

A tubular applicator 30 is releasably connected at a first end 32 to the collar 8 of an adhesive peristomal baseplate 1 to assist the user in positioning the baseplate 1 around a tracheostoma 100 on the user's neck. The applicator defines an internal flowpath 36 which is placed in fluid communication with the tracheostoma 100 via the collar 8 of the baseplate. The flowpath 36 opens outwardly via at least one outer aperture 34, 35 in the applicator 30. The at least one outer aperture 34, 35 has a total section area greater than a first section area A1 of the flowpath 36 at the first end 32 of the applicator.

## Description

This invention relates to adhesive peristomal baseplates for use in mounting a heat and moisture exhanger, speaking valve or other accessory in fluid communication with a tracheostoma, and in particular to the procedure of applying the baseplate to adhere to the peristomal skin of the user's neck.

A tracheostoma is an artificial opening formed through the neck into the trachea (windpipe), through which a person can breathe. A permanent tracheostoma may be formed, for example, when the upper airway is closed following laryngectomy or other surgery so that it is no longer possible to breathe through the nose or mouth. A person who breathes through a permanent tracheostoma may be referred to as a "neck breather".

One problem for neck breathers is how to maintain normal moisture in the lower airway in the absence of the upper airway which would normally regulate the temperature and humidity of the air flowing into the lungs. For this purpose a heat and moisture exhanger, often referred to as an HME, may be arranged in fluid communication with the tracheostoma. An HME commonly comprises a sponge impregnated with a hygroscopic salt, which acts as a filter and, by storing heat and moisture from the outbreath, warms and humidifies the inbreath.

Another problem is how to speak, which naturally would be accomplished by the outbreath passing through the larnyx (voicebox). For this purpose a tracheoesophageal puncture (fistula) may be created to house a small one-way valve, also referred to as a voice prosthesis, between the trachea and the esophagus. The voice prosthesis prevents flow from the esophagus into the trachea, while allowing a limited flow from the trachea into the esophagus which vibrates the remaining mucosa, producing sound which can be modulated into speech in the oral cavity.

In order to force the outbreath through the voice prosthesis to produce the voice, it is necessary to temporarily block the normal outflow through the tracheostoma. This is accomplished by a so-called speaking valve, which is arranged in fluid communication with the tracheostoma. In manually occluded speaking valves, the outbreath is blocked by pressing with a finger against a resiliently biased button. In automatic speaking valves the outbreath is blocked by increasing the pressure or flow rate of the outbreath or, in some models, of the inbreath.

An accessory such as an HME or speaking valve may be releasably mounted on a collar which in turn is sealingly connected in fluid communication with the tracheostoma, and which can remain in place while the accessory is removed and replaced at more frequent intervals, e.g. to change the filter or clean any accumulated secretions.

The collar can form one end of a stoma button, which is a short, flanged tube inserted into the stoma, or may form part of a peristomal baseplate.

A peristomal baseplate includes a flexible sheet which is adhesive on its inner, skin-facing side, usually with a release paper to protect the adhesive. A hole is formed in the sheet, and the collar surrounds the hole and extends away from the flexible sheet on its opposite, outer side.

The user peels off the release paper and then applies the adhesive side of the sheet to their peristomal skin (the skin of the neck surrounding the tracheostoma) so that the tracheostoma opens through the hole, with the adhesive providing a sealed fluid connection between the collar and the tracheostoma. The baseplate can remain in place for several days while one or more accessories are releasably and interchangeably attached to the collar.

Adhesive baseplates are also used, for example, to connect colostomy bags to intestinal stoma, and various applicators have been developed to help the user in managing their intestinal stoma.

For example, GB2425482A teaches a tubular applicator which is inserted into the central collar of an elasticated, non-adhesive barrier. The barrier is positioned over an intestinal stoma and then covered by the adhesive baseplate of a colostomy bag.

GB2109688A teaches an applicator system including a tubular band for supporting the elasticated neck of a colostomy bag during installation.

WO2019141324A1 teaches a tubular, pyramidal applicator for use in applying a temporary cover to an intestinal stoma.

In contrast to baseplates for intestinal stoma, a tracheostoma baseplate is used in a relatively less aggressive fluid environment, but must achieve an adhesive bond on peristomal skin that is often more sharply folded than that of the abdomen, and in the absence of any protruding stomal lip which is typically present in an intestinal stoma and helps in sealing. Accordingly, in order to achieve a reliable seal, a tracheostoma baseplate may be relatively thinner and more flexible than a baseplate for an intestinal stoma.

In use, the user may place the baseplate on a table (adhesive side up) while they remove the release paper, and then stand in front of a mirror while they position the baseplate around their tracheostoma. However, it can be difficult to pick up the baseplate and manipulate the flexible sheet into position on the neck without inadvertently folding and sticking the flexible sheet to itself, or contaminating the adhesive with finger marks or clothing fibres.

One technique is to insert a finger into the collar, which however risks contaminating the flowpath which leads to the lungs. This is undesirable since the collar is on the lung side of the HME filter which performs the normal function of the mucosa of the upper airway in trapping airborne pathogens and particles.

Users who have limited movement in their fingers may find it particularly difficult to avoid contaminating either the adhesive or the inside of the collar while simultaneously observing in a mirror the position of the tracheostoma through the hole in the baseplate, to ensure that the tracheostoma is not occluded by the adhesive sheet.

It is a general object of the present invention to make it easier for the user to position an adhesive peristomal baseplate around a tracheostoma.

Accordingly the present invention provides an applicator for use in applying an adhesive peristomal baseplate to a tracheostoma, as defined in the claims.

In a related aspect, the invention provides a tracheostoma care system including an applicator and an adhesive peristomal baseplate, as defined in the claims.

The novel applicator includes a body having a first end, a connection structure at the first end, and at least one outer aperture.

The applicator further defines a flowpath extending within the body and opening through the body at the first end and via the at least one outer aperture.

The connection structure is arranged to releasably connect the first end of the body to a collar of the baseplate to mount the baseplate on the applicator and to place the flowpath in fluid communication with the tracheostoma, via the collar, in a use position of the baseplate.

The flowpath defines a flow direction and has a first section area in a plane normal to the flow direction at the first end of the body, the at least one outer aperture having a total section area greater than the first section area of the flowpath.

The enlarged section area of the at least one outer aperture helps ensure that the flowpath remains open throughout the application procedure, so that the user can continue to breathe normally through the flowpath while holding the applicator which is engaged with the collar of the baseplate. The user may hold the applicator in one hand to mount the baseplate on the applicator, using the other hand to remove the release paper and then, after positioning the baseplate mounted on the applicator over the tracheostoma, to smooth the flexible sheet over the peristomal skin. The applicator is then detached from the collar of the baseplate and replaced by the required accessory.

Further features and advantages will be evident from the following illustrative embodiments which will now be described, purely by way of example and without limitation to the scope of the claims, and with reference to the accompanying drawings, in which:
Fig. 1 shows a first, conventional adhesive peristomal baseplate for use in connecting an accessory to a tracheostoma;
Fig. 2 is a section through the first baseplate at II - II of Fig. 1;
Fig. 3 shows a second, conventional adhesive peristomal baseplate for use in connecting an accessory to a tracheostoma;
Fig. 4 is a section through the second baseplate at IV - IV of Fig. 3;
Fig. 5 shows an accessory mounted on the first baseplate in its use position, in fluid communication with a tracheostoma;
Fig. 6 shows an applicator in accordance with an embodiment of the invention;
Fig. 7 shows the first baseplate mounted on the applicator ready for application to the tracheostoma;
Fig. 8 is a side view of the applicator;
Fig. 9 is a longitudinal section through the applicator, taken at IX - IX of Fig. 8;
Fig. 10 is another longitudinal section through the applicator, taken at X - X of Fig. 9;
Fig. 11 is a longitudinal section corresponding to Fig. 10 and showing a modification of the applicator;
Fig. 12 is an end view of the applicator, seen from the first end;
Fig. 13 is a cross-section through the applicator, taken at XIII - XIII of Fig. 8;
Fig. 14 shows the section of Fig. 10, illustrating the respective section areas of the lateral apertures and first and second end apertures;
Fig. 15 shows the section of Fig. 13, illustrating the curved plane P3 conforming to the periphery of one lateral aperture in which its section area is taken;
Figs. 16 and 17 show the accessory, respectively during connection to (Fig. 16) and disconnection from (Fig. 17) the first baseplate;
Figs. 18 and 19 show the first end region of the applicator, respectively during connection to (Fig. 18) and disconnection from (Fig. 19) the first baseplate; and
Figs. 20 and 21 show the first end region of the applicator in the modified configuration of Fig. 11, respectively during connection to (Fig. 20) and disconnection from (Fig. 21) the first baseplate.

Reference numerals and characters appearing in more than one of the figures indicate the same or corresponding elements in each of them.

### Baseplate and accessories

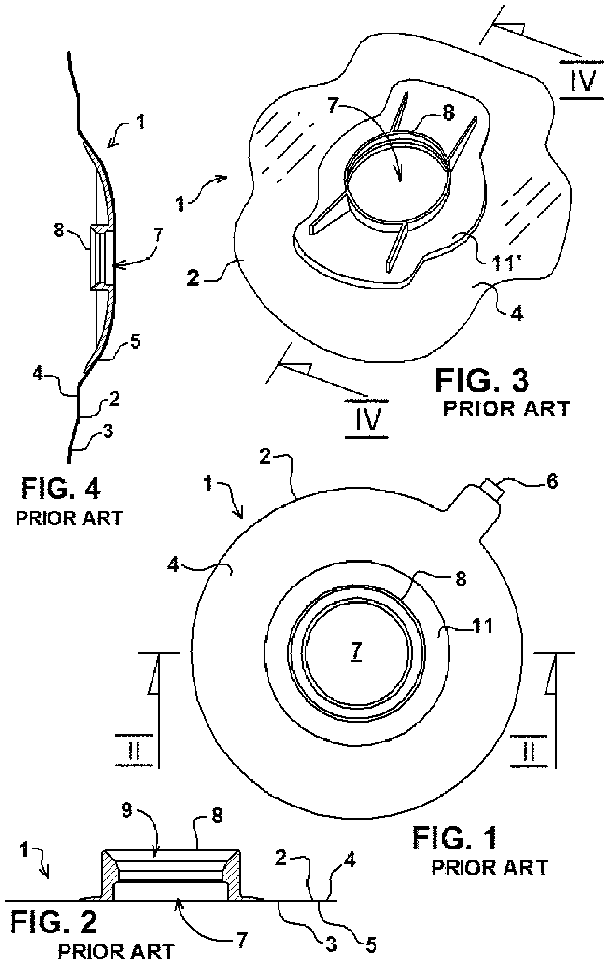

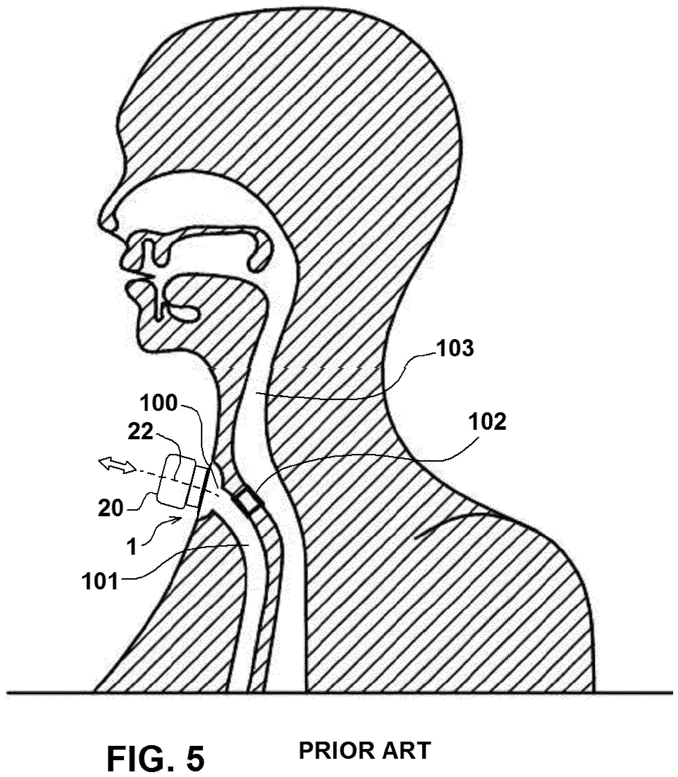

Referring to Figs. 1 - 5, each baseplate 1 includes a flexible sheet 2 having oppositely facing, first and second surfaces 3, 4. A stoma aperture 7 is formed in the sheet 2 to communicate with the tracheostoma 100 in the use position of the baseplate, as shown in Fig. 5. A collar 8 surrounds the stoma aperture and extends away from the second surface 4.

The first surface 3 is adhesive and may be covered by a release paper 5 which is peeled away by the user before applying the baseplate 1 to their tracheostoma - which is to say, before pressingly adhering the adhesive surface 3 to the peristomal skin surrounding the tracheostoma, to mount the baseplate 1 in its use position with the tracheostoma 100 exposed through the stoma aperture 7 and collar 8.

The release paper 5 may include a tab 6 to assist the user to grip it. The second surface 4 is not adhesive and may be flesh coloured, or the sheet 2 may be transparent, so as to be inconspicuous on the neck in use.

Other arrangements are possible; for example, the adhesive of the adhesive peristomal baseplate could be applied as a separate component between the flexible sheet and the peristomal skin.

The collar 8 may be moulded in a material that is relatively less flexible than the sheet 2, and may be bonded to the sheet 2, e.g. by welding at a peripheral flange 11, 11'. As illustrated by the first baseplate (Figs. 1 and 2), the sheet 2 may be generally circular with a circular flange 11. Alternatively, as illustrated by the second baseplate (Figs. 3 and 4), the sheet 2 and flange 11' may be shaped to define a major axis which can provide a more comfortable fit in a tracheostoma which is deeply recessed between the muscles of the neck.

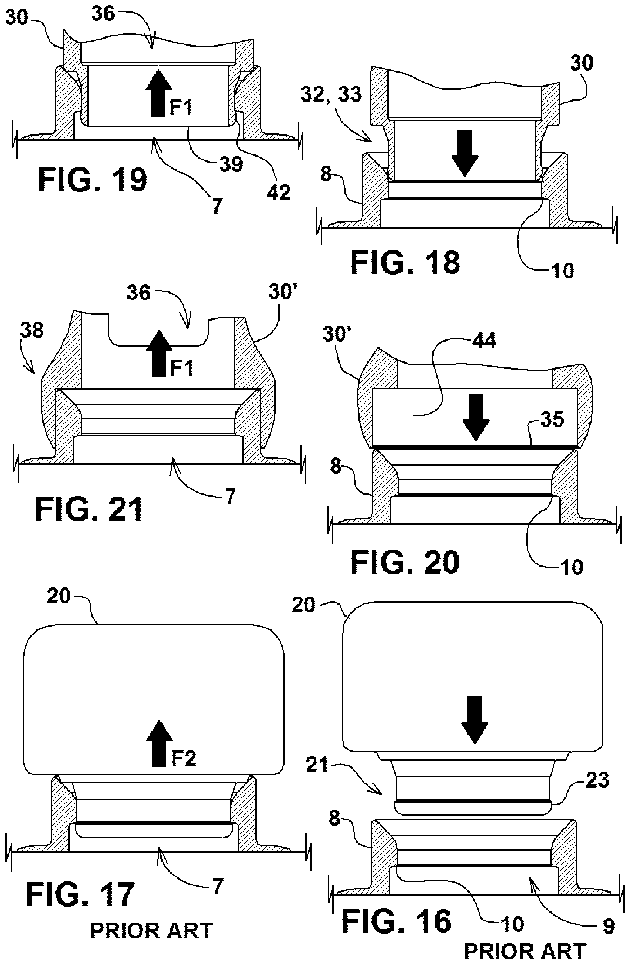

By way of example, the flexible sheet 2 could be made from a film, e.g. a polyurethane film, or could be a woven material. The adhesive could be for example a hydrocolloid (for greater comfort) or a relatively more tenacious adhesive for greater security, for example, a polyethane based adhesive such as commonly used on sticking plasters. The collar 8 could be injection moulded from a suitable plastics material such as ethylenevinyl acetate with a standardised internal diameter, for example, around 22.6mm. Referring also to Figs. 16 and 17, the collar 8 defines a connection structure 9 which is configured to releasably engage a corresponding connection structure 21 of an accessory 20. The accessory 20 defines an internal flowpath 22 and is releasably connectable to the connection structure 9 of the collar 8 to sealingly connect the internal flowpath 22 in fluid communication with the tracheostoma 100, via the collar 8 and stoma aperture 7.

As best seen in Figs. 16 and 17, the connection structure 21 of the accessory may be configured to be received inside the collar 8. As illustrated, the connection structure 21 may include a radially outwardly extending protuberance 23, which may be annular as shown, and which is received in snap-fit relation beneath a radially inwardly extending protuberance 10 of the connection stucture 9, which also may be annular as shown.

In this and other arrangements, the accessory 20 may be connectable to the collar 8 by pushing the accessory towards the collar (Fig. 16), and releasable from the collar by pulling the accessory 20 away from the collar 8 with a threshold release force, referred to below as the second release force F2 (Fig. 17). Either or both of the connection structures 9, 21 may flex as the protuberance 23 passes the protuberance 10 between the connected and disconnected positions.

In alternative arrangements (not shown), the collar 8 could be configured to be received inside the connection structure 21 of the accessory, in which case the collar could have a radially outwardly extending protuberance to engage a radially inwardly extending protuberance of the accessory. Other mechanical connection arrangements are possible.

The user may leave the baseplate in its use position for an extended period, perhaps several days, during which time a plurality of interchangeable accessories may be selectively connected and disconnected to and from the collar. For example, the user may disconnect the accessory for cleaning or replace it with another accessory with a fresh filter or a different flow rate to suit the activity pattern of the user.

The accessory 20 could be, for example, a heat and moisture exchanger (HME) or a speaking valve which is operable to selectively obstruct an outward airflow from the tracheostoma 100 through the internal flowpath 22 of the accessory. In either case, when the user is not speaking, the accessory 20 may allow a full, bidirectional airflow (both inbreath and outbreath) through its internal flowpath 22, which is necessary where the user does not have a patent (functional) upper airway, for example following a laryngectomy.

When the speaking valve closes, the outbreath is directed from the trachea 101 through a voice prosthesis 102 into the esophagus 103, producing the voice (Fig. 5). The speaking valve could be manually occluded, by manipulating a button or other mechanism (not shown), or automatically occluded by increasing the pressure or flow rate of the outbreath or inbreath above a threshold value. If the valve is closeable on the outbreath, it may include a pressure relief mechanism to open the internal flowpath if the user coughs, which may be manually reset to enable the user to speak again. The accessory could combine an HME, speaking valve, and/or other functional elements.

### Applicator

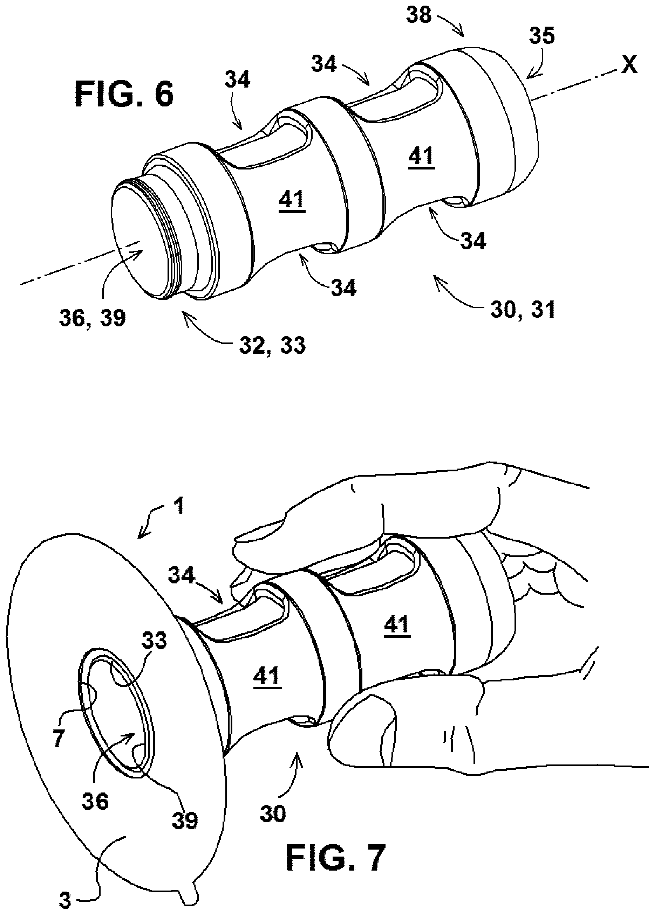

Referring now to Figs. 6 - 10, 12 and 13, the applicator 30 includes (and may consist of) a body 31 having a first end 32, a connection structure 33 at the first end 32, and at least one outer aperture 34, 35. The at least one outer aperture may include one or more lateral apertures 34, or a second end aperture 35, or a combination thereof, as illustrated and further explained below.

The interior space of the body 31 defines a flowpath 36 which extends within the body 31 and opens through the body 31 at the first end 32 and via the at least one outer aperture 34, 35.

As illustrated, the body may define a wall 37 which extends around the flowpath 36 along a length axis X between the first end 32 and an opposite, second end 38 of the body. The wall 37 may define a generally cylindrical shape of the body. The body 31 may have an overall length along its length axis X greater than about two times its overall external diameter or maximum transverse dimension relative to the length axis X.

The flowpath 36 may open through the body 31 via a first end aperture 39 at the first end 32 and a second end aperture 35 at the second end, wherein the length axis X passes (optionally, centrally, as illustrated) through each of the first end aperture 39 and the second end aperture 35.

This enables the user to view the tracheostoma in a mirror, via the aligned end apertures 35, 39, as s/he applies the baseplate to the tracheostoma, so as to ensure that the sticky sheet 2 does not occlude the tracheostoma.

The aligned end apertures 35, 39 also make it possible to injection mould the body 31 in a relatively simple mould, having a core which defines the flowpath 36 and which is withdrawn via the larger, second end aperture 35. For this purpose the flowpath 36 may be defined by an internal surface of the body having a diameter which remains constant or increases in only one direction along the length axis X, e.g. step-wise as illustrated. The body may be generally tubular, and the internal surface may be a generally cylindrical surface of revolution about the length axis X, as illustrated.

If a plurality of lateral apertures 34 are provided, as further discussed below, then they may be arranged in diametrically opposed relation, e.g. as illustrated, so that they can be formed by two outer mould parts which are separated to release the moulding, the mould thus comprising only three parts.

In this and other arrangements, the body 31 may be transparent or translucent in order to improve visibility of the tracheostoma. For example, the body 31 may be moulded in a transparent or translucent plastics material, e.g. a polyolefin, e.g. medical grade polypropylene.

Alternatively or additionally to the second end aperture 35, the at least one outer aperture may include at least one lateral aperture 34, optionally a plurality of lateral apertures 34, which are formed in the wall 37 between the first and second ends 32, 38 of the body.

Optionally, as illustrated, each lateral aperture 34 may be defined by a closed or continuous periphery 40 which defines a window in the wall 37.

Where a plurality of lateral apertures are provided, they may be arranged in diametrically opposed pairs which are spaced apart angularly around the length axis, and/or spaced apart axially along the length axis X. As illustrated, two or more groups of lateral apertures 34 may be provided, the apertures 34 of each group being spaced apart angularly around the length axis, the groups being spaced apart axially along the length axis X.

### Flowpath section area

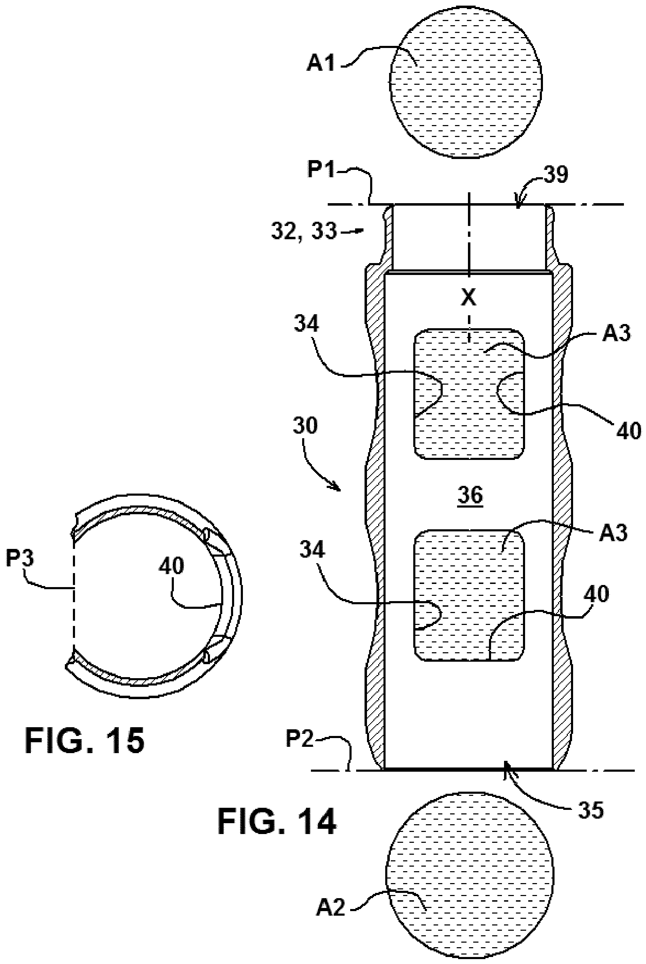

Referring now to Figs. 14 and 15, the flowpath 36 defines a flow direction at the first end 32 of the body 31. In the illustrated embodiment, the flow direction is axially through the first end aperture 39 in either direction of the length axis X.

The flowpath 36 has a first section area A1 in a plane P1 normal to the flow direction (length axis X) at the first end 32 of the body 31. The plane P1 may contain the periphery of the first end aperture 39 so that the first section area A1 is defined by the radially inwardly facing surface of the body 31 at the first end aperture 39, as shown.

For ease of illustration, the first section area A1 is shown in Fig. 14 as seen in end view (looking along the length axis X) in proportion alongside the longitudinal section through the body 31.

The applicator 30 may be connected in fluidly sealed relation to the collar 8 of the baseplate 1, so that in the use position of the baseplate as shown in Fig. 5, after sealingly attaching the flexible sheet 2 to the peristomal skin, but before detaching the applicator 30 from the collar 8, the flowpath 36 of the applicator is sealingly connected in fluid communication with the tracheostoma 100 via the collar 8.

Thus, when the baseplate 1 is adhesively secured to the peristomal skin all the way around the tracheostoma, the user must breathe exclusively through the flowpath 36. It is important therefore to ensure that the user can continue to breathe normally through the flowpath 36 during the application procedure and until the applicator 30 is removed from the collar 8.

For this purpose, the at least one outer aperture 34, 35 has a total section area greater than the first section area A1 of the flowpath 36.

The relatively enlarged total section area of the at least one outer aperture 34, 35 compared with the first section area A1 of the flowpath 36 helps to ensure that the minimum section area of the flowpath 36 is not reduced to a value less than its first section area A1 at the first end 32, in the event that the at least one outer aperture should be partially occluded by the user's fingers when gripping the applicator 30 in use.

Thus, the user can continue to breathe normally through the flowpath 36 while gripping the applicator 30.

This can be further ensured by further enlarging the total section area of the at least one outer aperture 34, 35.

Preferably therefore, the at least one outer aperture 34, 35 has a total section area at least 50% greater than the first section area A1 of the flowpath - which is to say, the total section area of the at least one outer aperture is not less than 150% of the first section area A1 of the flowpath 36.

More preferably, the at least one outer aperture 34, 35 has a total section area at least 100%, yet more preferably 150%, most preferably 200% greater than the first section area A1 of the flowpath - which is to say, the total section area of the at least one outer aperture is not less than 200%, yet more preferably 250%, most preferably 300% of the first section area A1 of the flowpath 36.

Further advantageously, as shown, the total section area of the at least one outer aperture can be more than 300%, more than 400%, or even more than 500% of the first section area A1 of the flowpath 36.

In the illustrated embodiment there are four lateral apertures 34, each having a section area A3, so that the total section area of the at least one outer aperture 34, 35 is equal to (4·A3)+A2, wherein A2 is the section area of the second end aperture 35.

In the illustrated embodiment, the periphery of the second end aperture 35 lies in a plane P2 parallel with the plane P1 and normal to the length axis X, which passes centrally through the axially aligned, first and second end apertures 39, 35. Thus, the section area A2 of the second end aperture 35 is defined in the plane P2, and is illustrated in Fig. 14 in a similar way to the first section area A1 of the first end aperture 39.

In alternative embodiments, it is possible however that the periphery of the second end aperture 35 could extend in three dimensions, in which case its section area A2 will be defined by a surface of least possible area which is bounded by the entire periphery.

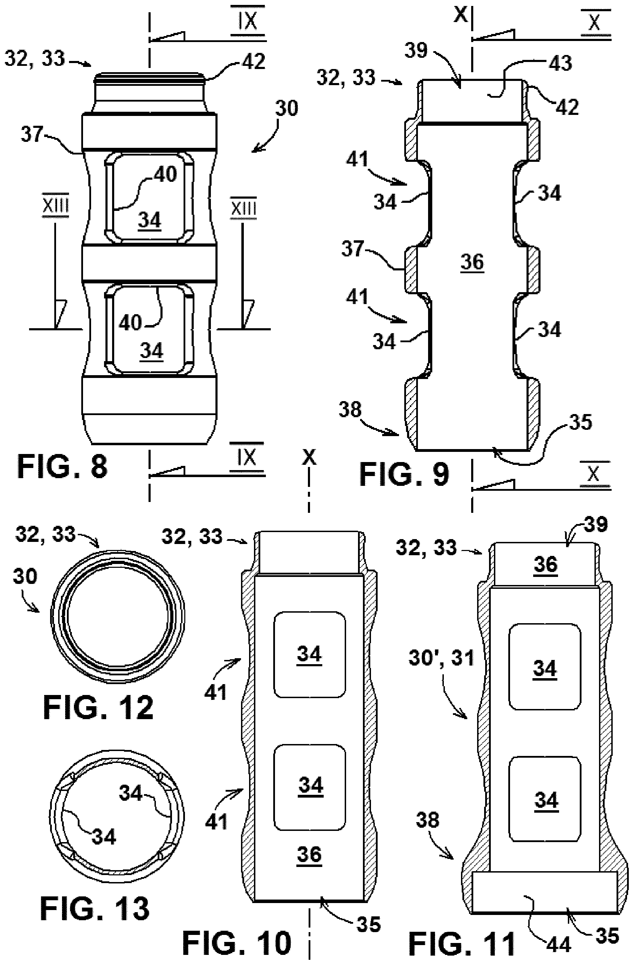

Fig. 15 illustrates how the section area A3 of each lateral aperture 34 is defined as the area of a surface of least possible area bounded by the entire periphery 40 of the lateral aperture 34. This imaginary surface will be curved, at least at the axial ends of the aperture 34, but for rough comparison may be approximated by a straight line P3 in the plane of the section of Fig. 15. This section is the same as that of Fig. 13, being taken at XIII - XIII of Fig. 8 and cutting through the axial centre of the aperture 34.

If an outer aperture is formed (not shown) with an open rather than closed periphery, then the section area of the aperture may be calculated based on a closed periphery defined by closing the open portion of the periphery with an imaginary straight line.

Advantageously, the at least one outer aperture may extend in three dimensions of the body 31 of the applicator. This makes it yet more difficult to inadvertently block the entire outer aperture, for example, with a finger or thumb. As illustrated, by way of example, this can be accomplished by shaping the periphery 40 of the lateral aperture 34 to extend both along and around the length axis X of the body 31.

Fig. 7 shows how the baseplate 1 is mounted in use on the applicator 30, which is used to position the baseplate on the user's neck in its use position as shown in Fig. 5.

As illustrated, the wall 37 of the body 31 may have at least one region 41 which is recessed radially inwardly (at least at its radially outwardly facing surface) with respect to the length axis X, and which defines at least a part of a (closed or open) periphery 40 of the at least one lateral aperture 34 formed in the wall. The or each radially inwardly recessed region 41 may define an annular region of reduced external diameter which extends around the length axis X. For example, as in the illustrated embodiment, two radially inwardly recessed regions 41 may be spaced apart axially along the length axis X.

As shown in Fig. 7, if the user grips the applicator 30 by placing the fingers over the lateral apertures 34, as shown, the user's fingers are supported by the larger diameter regions in-between these recessed regions 41. The radially inwardly recessed regions 41 of the wall 37 thus provide a pathway through which air can continue to flow through the lateral apertures 34 beneath the region of the grip, and thus further help to ensure that the lateral apertures 34 remain unobstructed in use.

### Method of use

The applicator 30 may be re-usable multiple times, and may be supplied separately for use with any compatible baseplates, or as part of a tracheostoma care system including one or more adhesive peristomal baseplates of any compatible type, optionally also one or more accessories such as an HME, speaking valve, cover, connector, or other accessories or combinations thereof.

Referring again to Figs. 5 and 7, when the user wishes to apply a new baseplate 1 s/he first mounts the baseplate 1 on the first end 32 of the applicator. Conveniently, the release paper can be left on the baseplate while the baseplate is engaged with the applicator, e.g. by placing the baseplate with its release paper side on a table or on the palm of one hand, while engaging the applicator 30 with the collar 8.

With the baseplate mounted on the applicator, the user can then remove the release paper 5 and then, holding the applicator 30 to avoid touching the baseplate 1, apply the sticky side 3 of the baseplate 1 to their tracheostoma 100. By arranging a mirror at a suitable angle the user can observe the position of the tracheostoma 100 through the second end aperture 35 while positioning the baseplate so that the tracheostoma 100 is completely exposed through the stoma aperture 7. Using the fingers of the other hand, the user can then smooth out the flexible sheet 2 to provide an airtight, adhesive seal to the peristomal skin. Once the baseplate 1 is in its use position as shown in Fig. 5, the user can detach the applicator 30 from the collar 8 and replace it with the required accessory 20. If convenient, the user may disconnect the applicator 30 before finishing smoothing out the flexible sheet 2 into its final position.

The applicator may also be used in a similar way by a healthcare professional to apply the baseplate to a patient's tracheostoma.

### Connection structure

Referring now to Figs. 18 and 19, the connection structure 33 of the body 31 is arranged to releasably connect the first end 32 of the body 31 to the collar 8 of the baseplate 1 to mount the baseplate 1 on the applicator 30.

The connection structure of the body may be connectable to the same connection structure 9 as the accessory 20, or alternatively may engage a different structure of the collar of the baseplate from that which retains the accessory 20. In either case, the connection structure of the body may be arranged to releasably connect the first end of the body to the collar of the baseplate when the collar is received inside the connection structure of the body, or alternatively, when the connection structure of the body is received inside the collar of the baseplate.

By connecting the connection structure 33 of the body 31 inside the collar 8, where the collar 8 is arranged to receive the connection structure 21 of the accessory 20 inside the collar, the connection structure 33 can engage the same connection structure 9 as the accessory 20. This provides a simple connection and allows the external surface of the collar 8 to be adapted, e.g. with stiffening ribs or other structures as illustrated in Figs. 3 and 4.

Alternatively, by connecting the connection structure of the body radially outside the collar of the baseplate, it may be ensured that any contamination of the connection structure of the body will not transfer to the flowpath through the collar of the baseplate.

Whether connected inside or outside the collar 8, and whether to the same or a different connection structure of the collar to that which retains the accessory 20, the connection structure of the body may be connectable to the collar of the baseplate by pushing the applicator towards the collar, preferably in the direction of the length axis X, as illustrated in Fig. 18, and releasable from the collar of the baseplate by pulling the applicator away from the collar with a first release force F1, as illustrated in Fig. 19.

The second release force F2 required to release the accessory 20 may be greater than the first release force F1 required to release the applicator 30, so that the accessory 20 is not releasable from the collar 8 by pulling the accessory 20 away from the collar with only the first release force F1. This makes it easier to remove the applicator, optionally before the baseplate is fully adhered to the peristomal skin, so that the user can finish smoothing out the flexible sheet after removing the applicator.

By way of example, the second release force F2 could be around 10N - 40N, for example, about 25N, with the first release force F1 being half that value.

Optionally, the push-fit connection may be a snap-fit connection. For this purpose, where as illustrated the connection structure 33 of the body 31 is received inside the collar 8, the connection structure 33 may include a radially outwardly extending protuberance 42, which may be annular as shown, and which is received in snap-fit relation beneath the radially inwardly extending protuberance 10 of the connection structure 9 of the collar 8. Either or both of the connection structures 9, 33 may flex as the protuberance 42 passes the protuberance 10 between the connected and disconnected positions, in a similar way to the connection and disconnection of the accessory 20 as described above with reference to Figs. 16 and 17.

The (or each) connection structure of the applicator (and/or the connection structure of the collar) may be a continuous annular structure, or could be discontinuous around the length axis X, e.g. by dividing the connection structure by radial discontinuities or slits into a plurality of fingers that are angularly spaced around the length axis, like a split collet, so that the fingers can flex slightly radially relative to one another.

As illustrated, the protuberance 42 may be slightly less prominent or less abruptly angled than the corresponding protuberance 23 of the connection structure 21 of the accessory 20, so that it releases at the relatively lower, first release force F1.

The applicator may be configured with more than one connection structure to engage differently shaped or sized collars of different baseplates. Additionally, or alternatively, the connection structure (or another connection structure) of the applicator may be arranged to grip frictionally rather than in snap-fit relation, e.g. by providing a smooth surface which slightly interferes with the surface of the collar of the baseplate. For example, either or both of the surfaces could be cylindrical or slightly tapering, or one could be cylindrical and the other tapering.

Where the applicator is configured to engage the radially outward surface of the collar 8, e.g. of the first baseplate of Figs. 1 and 2, the collar 8 may be received in a connection structure defined by a radially inward surface of the applicator 30, which may define a part of the flowpath 36.

This radially inward surface could be the radially inwardly facing surface 43 of the first end 32 which defines another connection structure 33 on its radially outward surface, as illustrated. In this way, the applicator 30 may be arranged to grip larger and smaller collars 8 at the same, first end 32.

Referring now to Fig. 11 and Figs. 20 and 21, in another possible arrangement, the second end 38 of the body 31 may be adapted to provide yet another connection structure, which as illustrated may be another radially inwardly facing surface 44 defining the outward end of the flowpath 36 at the second end aperture 35. The flowpath 36 and second end aperture 35 may be radially enlarged in this region, relative to the rest of the flowpath 36, so that the surface 44 can engage a collar of larger diameter than that which is engaged by the first end 32 of the adapted applicator 30', which otherwise is the same as the applicator 30. Yet another external connection structure (not shown) could be arranged at the second end 38 so that four different sizes of collar can be mounted on the same applicator.

Figs. 20 and 21 show how the enlarged surface 44 can frictionally engage the collar 8 as a slight interference fit, which like the connection structure 33 at the first end 32 can provide a release force F1 that is lower than the release force F2 of the accessory 20.

### Summary

In summary, embodiments provide a tubular applicator 30 which is releasably connected at a first end 32 to the collar 8 of an adhesive peristomal baseplate 1 to assist the user in positioning the baseplate 1 around a tracheostoma 100 on the user's neck. The applicator defines an internal flowpath 36 which is placed in fluid communication with the tracheostoma 100 via the collar 8 of the baseplate. The flowpath 36 opens outwardly via at least one outer aperture 34, 35 in the applicator 30. The at least one outer aperture 34, 35 has a total section area greater than a first section area A1 of the flowpath 36 at the first end 32 of the applicator.

The body 31 may be of generally cylindrical shape as shown in the illustrated embodiments, but alternatively could have a different shape, e.g. widening outwardly away from the first end 32. The body 31 may define an outer surface which conforms generally to a surface of rotation about its length axis X, or alternatively could be polygonal or elliptical or any other shape that is comfortable for the user.

In alternative embodiments, the lateral aperture or apertures could be arranged other than in diametrically opposed relation. For example, three apertures or three sets of apertures could be equally angularly spaced around the length axis, hence centred at 120 degree intervals.

In further alternative embodiments, the wall of the body could be arranged to define one or more lateral apertures which are not enclosed within a closed or continuous periphery 40, but open at one side or end. For example, the wall of the body could be configured as one or more spirally curved elements which extend angularly around and axially along the length axis X of the body, with the lateral apertures formed between the spiral elements and unbounded at the second end 38 of the body.

In yet further alternative embodiments, the wall of the body could be configured in a skeletal form, e.g. as a lattice, framework or mesh, wherein the total section area of the lateral aperture or apertures is greater than that of the skeletal, solid parts of the wall.

Instead of being re-usable, the applicator could be designed to be disposable, and may not be re-usable (e.g. having a part that is damaged or otherwise disabled in use, to prevent re-use).

Many further adaptations are possible within the scope of the claims.

In the claims, reference numerals and characters are provided in parentheses, purely for ease of reference, and should not be construed as limiting features.

## Claims

1. An applicator (30, 30') for use in applying an adhesive peristomal baseplate (1) to a tracheostoma (100), the applicator (30) including:
a body (31) having
a first end (32),
a connection structure (33, 43) at the first end, and
at least one outer aperture (34, 35); and
a flowpath (36) extending within the body and opening through the body at the first end and via the at least one outer aperture;
the connection structure (33) being arranged to releasably connect the first end of the body to a collar (8) of the baseplate to mount the baseplate on the applicator and to place the flowpath in fluid communication with the tracheostoma, via the collar, in a use position of the baseplate;
the flowpath (36) defining a flow direction and having a first section area (A1) in a plane (P1) normal to the flow direction at the first end (32) of the body;
the at least one outer aperture (34, 35) having a total section area greater than the first section area of the flowpath.

2. An applicator according to claim 1, wherein the body defines a wall (37) extending around the flowpath along a length axis (X) between the first end (32) and an opposite, second end (38) of the body;
the flowpath opening through the body via a first end aperture (39) at the first end,
the at least one outer aperture including a second end aperture (35) at the second end of the body,
the length axis passing through the first end aperture and the second end aperture.

3. An applicator according to claim 1, wherein the body defines a wall (37) extending around the flowpath along a length axis (X) between the first end (32) and an opposite, second end (38) of the body, and the at least one outer aperture includes at least one lateral aperture (34) formed in the wall (37) between the first and second ends of the body.

4. An applicator according to claim 3, wherein the wall (37) has at least one region (41) which is recessed radially inwardly with respect to the length axis (X), the at least one radially inwardly recessed region (41) defining at least a part of a periphery (40) of the at least one lateral aperture (34) formed in the wall.

5. An applicator according to claim 3 or claim 4, wherein the at least one outer aperture includes a plurality of lateral apertures (34) formed in the wall (37) between the first and second ends of the body.

6. An applicator according to claim 3, claim 4 or claim 5, wherein the flowpath opens through the body via a first end aperture (39) at the first end (32),
the at least one outer aperture including a second end aperture (35) at the second end (38) of the body,
the length axis (X) passing through the first end aperture and the second end aperture.

7. An applicator according to claim 1, wherein the at least one outer aperture has a total section area at least 50% greater than the first section area (A1) of the flowpath.

8. An applicator according to claim 1, wherein the at least one outer aperture extends in three dimensions of the body (31).

9. An applicator according to claim 1, wherein the connection structure (43, 44) is arranged to releasably connect the first end of the body (31) to the collar of the baseplate when the collar is received inside the connection structure (43, 44).

10. An applicator according to claim 1, wherein the connection structure (33) is arranged to releasably connect the first end (32) of the body (31) to the collar (8) of the baseplate (1) when the connection structure (33) is received inside the collar (8).

11. A tracheostoma care system including an applicator (30, 30') in accordance with any preceding claim, and an adhesive peristomal baseplate (1);
the baseplate (1) including a flexible sheet (2) having:
oppositely facing, first and second surfaces (3, 4), the first surface (3) being adhesive;
a stoma aperture (7) for communicating with the tracheostoma (100) in a use position of the baseplate; and
a collar (8) surrounding the stoma aperture (7) and extending away from the second surface (4);
the connection structure (33, 43) being arranged to releasably connect the first end (32) of the body (31) to the collar (8) of the baseplate (1) to mount the baseplate on the applicator and to place the flowpath (36) in fluid communication with the tracheostoma (100), via the collar (8), in the use position of the baseplate (1).

12. A tracheostoma care system according to claim 11, wherein the connection structure (33, 43) is connectable to the collar (8) by pushing the applicator (30, 30') towards the collar (8), and releasable from the collar by pulling the applicator away from the collar.

13. A tracheostoma care system according to claim 11, further including at least one accessory (20), the accessory defining an internal flowpath (22) and being releasably connectable to the collar (8) of the baseplate (1) to place the internal flowpath (22) of the accessory in fluid communication with the tracheostoma (100), via the collar (8), in the use position of the baseplate (1).

14. A tracheostoma care system according to claim 13, wherein the at least one accessory (20) includes at least one of:
a heat and moisture exchanger, and
a speaking valve which is operable to obstruct an outward airflow from the tracheostoma (100) through the internal flowpath (22) of the accessory.

15. A tracheostoma care system according to claim 13 or claim 14, wherein the connection structure (33, 43) is connectable to the collar (8) by pushing the applicator (30, 30') towards the collar (8), and releasable from the collar by pulling the applicator away from the collar with a first release force (F1); and
the at least one accessory (20) is connectable to the collar by pushing the accessory towards the collar, and releasable from the collar by pulling the accessory away from the collar with a second release force (F2);
the second release force (F2) being greater than the first release force (F1), whereby the at least one accessory (20) is not releasable from the collar (8) by pulling the accessory away from the collar with the first release force (F1).
